# EUROPEAN PATENT SPECIFICATION

(11) **EP 1 089 727 B1**
(45) Date of publication and mention of the grant of the patent: **07.12.2005**
(21) Application number: 99931721.7
(22) Date of filing: 17.06.1999
(51) Int. Cl.: A61K 31/255, A61K 9/127, A61P 35/00

(54) **LIPOSOMAL FORMULATIONS OF BUSULPHAN**
LIPOSOMFORMULATIONEN MIT BUSULPHAN
PREPARATIONS A BASE DE BUSULPHAN ENCAPSULEES DANS DES LIPOSOMES

(30) Priority: 18.06.1998 SE 9802197; 18.06.1998 US 89759 P
(43) Date of publication of application: 11.04.2001
(73) Proprietor: Hassan, Moustapha, Dr., 141 44 Huddinge (SE); Hassan, Zusana, 141 44 Huddinge (SE)
(72) Inventor: HASSAN, Moustapha, S-129 36 Hägersten (SE)
(74) Representative: Nyberg, Bengt
(86) International application number: PCT/SE1999/001093
(87) International publication number: WO 1999/065481

(56) References cited:
- EP-A1- 0 546 951
- WO-A1-88/06442
- WO-A1-92/22298
- WO-A1-95/08991

## Description

### FIELD OF THE INVENTION

This invention relates to the fields of biochemistry and medicine, and in particular to novel liposomal formulations. More specifically it relates to liposomal formulations of busulphan and to their process of manufacture. The present invention overcomes the insolubility problem of the drug when administered parenterally and decreases the toxic side effects of busulphan as a drug.

### BACKGROUND OF THE INVENTION

Busulphan (1,4-bis-(methanesulphonoxy)butane) is a bifunctional alkylating agent with potent antitumor effects. It has been widely used for treatment of malignant diseases, especially hematologic malignancies and myeloproliferative disorders. Its use was for a long time restricted to low dose oral therapy, with recorded side effects such as busulphan-induced pulmonary fibrosis (Oakhill et al. 1981. J. Clin.Pathol. 34(5):495-500.) and irreversible myelo-suppression (Canellos 1985. Chronic Leukemias In: Cancer: Principles and Practice of Oncology, pp 1739-1752). In 1983 was high-dose combination chemotherapy based on oral busulphan for pretransplant-conditioning of patients undergoing both autologous and allogeneic bone marrow transplantation (Santos, G.W. et al. 1983. N. Engl. J. Med. 309:1347-1353; Lu, C. et al. 1984. Cancer Treatm. Repts. 68:711-717) introduced. Since then, high dose busulphan replaces total body irradiation (TBI), most commonly in combination with cyclophosphamide, and has proven to be a most effective anti-leukemic regimen when used in conjunction with autologous or allogeneic hematopoietic stem cell support. The main advantage of high-dose busulphan therapy for use in marrow ablation treatment is that total body irradiation regimen is avoided, which is especially advantageous for young children and adults who received TBI as part of their initial therapy.

Oral busulphan in combination with cyclophosphamide has several serious side effects. Reported side effects are fatal liver failure (Miller, C. et al. 1991. Blood 78:1155), neurological disturbances like grand mal seizures, venoocclusive disease, severe nausea and vomiting (Vassal, G. et al. 1990. Cancer-Res. 50:6203-6207) as well as side effects in the lungs, such as interstitial pneumonia (Bandini, G. et al. 1994. Bone Marrow Transpl. 13:577-581).

Therefore attempts have been made to improve the clinical utility of the busulphan drug by providing a busulphan formulation for parenteral administration. U.S. patents 5,430,057 and 5,559,148 disclose physiologically acceptable solutions of busulphan, wherein N'N-dimethylacetamide, polyethylene glycol, propylene glycol, glycerin cyclodextrin, hydroxypropylbetacyclodextrin are used as solvents. In two other studies busulphan was dissolved in dimethylsulfoxide (DMSO) (Bhagwatwar H.P, Phadungpojna S, Chow D.S,Andersson B.S. 1996. Cancer Chemother Pharmacol 37:401) and dimethylacetamide (DMA) (Ehninger G, Schuler U, Renner U, Ehrsam M, Zeller K.P, Blanz J, Storb R, Deeg H.J. 1995. Blood 85:3247). However, despite the very promising results, these organic solvents have their own toxicity (Kennedy Jr G.L, Sherman H. 1986. Drug and Chemical Toxicology 9:147; Kinney L.A, Burgess B.A, Stula E.F, Kennedy Jr G.L. 1993. Drug and Chemical Toxicology 16:175; Malley L.A, Slone Jr T.W, Makovec G.T, Elliott G.S, Kennedy Jr G.L. 1995. Fundam Appl Toxicol 28:80; Martino M, Morabito F, Messina G, Irrera G, Pucci G, Iacopino P. 1996. Haematologica 81:59; Sperling S, Larsen I.G. 1979. Acta Ophthalmol (Copenh) 57:891; Yellowlees P, Greenfield C, McIntyre N. 1980. Lancet 2:1004). Further, it seems as if DMSO activates the metabolism of busulphan in the liver, resulting in reduced concentrations of busulphan in the blood of the patient after a few days regimen (Shuler et al. 1997. Abstract EBMT-meeting, Chamonix, France). The Spartaject™ technology has tried to achieve a formulation of busulphan for parenteral administration by forming microcrystals of busulphan coated with lecitin (or other phosopholipid) dissolved in mannitol and water. The size of these microcrystals are 0.1u. The main problems with this formulation is precipitation of the microcrystals during infusion of the drug. Also nothing is known about their biodistribution. Both busulphan dissolved in organic solvents and busulphan of microcrystalline form cross the blood brain barrier, giving rise to central nerve system toxicity.

A liposome is a completely closed lipid bilayer membrane which defines a closed aqueous compartment. Liposomes are microscopic delivery vesicles made, in part, from phospholipids which form closed, fluid filled spheres when mixed with water. Phospholipid molecules are polar, having a hydrophilic ionizable head, and a hydrophobic tail consisting of long fatty acid chains. Thus when sufficient phospholipid molecules are present with water, the tails spontaneously associate to exclude water while the hydrophobic phosphate heads interact with water. Liposomes may be either unilamellar, comprised of one lipid bilayer membrane, or bilayer liposomes, comprised of two layers of lipids. In the latter liposomes the outer layer of lipid molecules are oriented with their hydrophilic head portions towards the external aqueous medium and their hydrophobic tails pointed inwards towards the interior of the liposome. The inner layer of lipids lays directly beneath the outer layer; the lipids are oriented with their heads facing the aqueous interior of the liposome and their tails towards the tails of the outer lipid layer. Multilamellar liposomes are composed of more than one lipid bilayer membrane, which define more than one closed compartment and arc concentrically arranged.

Since the chemical composition of many drugs precludes their intravenous administration, liposomes can be very useful in adapting these drugs for intravenous delivery. Many hydrophobic drugs, including busulphan, fall into this category because they cannot be easily dissolved in a water-based medium and must be dissolved in alcohols or surfactants which have been shown to cause toxic reactions in vivo. Liposomes, composed of predominantly lipids, with or without cholesterol, are nontoxic. Further, liposomes have the potential of providing a controlled release of the administered drug over an extended period of time, and of reducing toxic side effects of the drug, by limiting the concentration of the free drug in the bloodstream. Liposomes can also alter the tissue distribution and uptake of drugs, and the altered tissue distribution can significantly increase the therapeutic effectiveness of the drug. Liposome/drug compositions can for these reasons increase the convenience of therapy by allowing higher drug dosage and less frequent drug administration.

The original liposome preparation of Bangham et al. (J. Mol. Biol., 1965, 13238-252) involves suspending phospholipids in an organic solvent which is then evaporated to dryness leaving a phospholipid film or powder. Next, hydration is performed, i.e. an aqueous phase is added to the phospholipid film and the mixture is dispersed by mechanical means resulting in liposomes consisting of multilamellar vesicles (MLVs). This preparation provides the basis for the development of small sonicated unilamellar vesicles (SUVs) described by Papahadjopoulos et al. (Biochim. Biophys. Acta. 1967. 135:624-638), and large unilamellar vesicles (LUVs).

A variety of sterols and their water soluble derivatives such as cholesterol hemisuccinate as well as a variety of tocopherols and their water soluble derivatives have been used to form liposomes. Liposomes themselves have been reported to have no significant toxicities in previous human clinical trials where they have been given intravenously.

Due to the difficulties associated with the solubility of busulphan a therapeutically effective liposome-encapsulated busulphan product has not been commercially available. This invention provides such a product that is stable, water soluble, non-toxic and still maintain activity.

### SUMMARY OF THE INVENTION

This invention provides, for the first time, a liposome-encapsulated busulphan. The formulations give water soluble, highly stable, pharmacologically active busulphan. Liposome-encapsulated busulphan overcomes the insolubility problem of the busulphan drug when administered intravenously. The main advantage of liposome-encapsulated busulphan is that the tissue distribution of the drug to the bone marrow and spleen is increased. Since these are the target organs for the therapy this results in increased therapeutic effectiveness and reduced toxic side effects of the drug. Further there is no accumulation of the drug in the liver or other organs known to be susceptible to busulphan toxicity. Liposome encapsulated busulphan according to the invention has with good result been administered to humans (see Example 8).

### DETAILED DESCRIPTION OF THE INVENTION

The present invention provides water soluble, highly stable, pharmacologically active busulphan by stabilizing the busulphan drug in liposomes having a size from 50 to 300 nm. The liposomes are comprised of lipids such as phosphatidylcholine, phosphatidylserine, glycero-3-phosphate, cholesterol, cardiolipin, stearoylamine, dicetylphosphate and phosphatidylglycerol. The liposomes may additionally comprise alpha-tocopherol. In the most preferred embodiment the liposomes comprise L-α-Phosphatidylcholine, 1,2-Dioleolyl-sn-Glycero-3-phosphate and cholesterol in a molar ratio of about 10: 1:10. In other preferred embodiments the liposomes comprise phosphatidylserine, phosphatidylcholine and cholesterol in a molar ratio of about 3:7: 10, or cardiolipin, phosphatidylcholine and cholesterol in a molar ratio of about 1:4:1.5, or phosphatidylcholine, cholesterol and stearoylamine in a molar ratio of about 7:1:1. In all four of these compositions, the content of each lipid may vary within approximately 30%.

During preparation of the liposomes organic solvents may be used to dissolve the lipids. Suitable solvents include, but are not limited to, chloroform, methanol, dimethylsulfoxide (DMSO), methylene chloride, ether, acetone and solvent mixtures such as benzene-methanol. In a preferred embodiment chloroform is used. Busulphan is added at this stage and the mixture is dried by evaporation, or other means, to a thin film.

The mixture can then be hydrated with e.g. glucose, sodium chloride, dextran, mannitol, ringer acetate, or sodium bicarbonate. The internal pH of the internal environment of the liposomes may be reduced to pH 2-6, which prevents hydrolysis. Phosphate, acetate, or citrate buffers may be used to adjust pH.

The liposome suspension may be annealed for 20-30 minutes at -60 to -80°C. Several methods may be used to form the liposomes of the invention, e.g. through vortexing or shaking. Multilamellar vesicles (MLVs) (0.05-5µM), stable plurilamellar vesicles SPLVs) or reverse phase evaporation vesicles (REVs) may be used. Preferably, MLVs are extruded through filters forming LUVs of sizes depending on the filter pore size. Polycarbonate filters of 50 to 300 nm pore sizes can be used. The liposomes are small unilamellar vesicles (SUV), rather than multilamellar vesicles (MLV). However both SUVs and MLVs are within the scope of the innovation. The liposomes are about 50-300 nm in size.

The busulphan-containing liposomes according the invention contain about 10 to 75 mg/ mL lipid. They have a drug (busulphan) to lipid molar ratio of about 1:2-1:140. Once the liposomes have been hydrated, they can be stored at 4°C for 20 days (see Example 1). To prolong the shelf life the liposomes can be dehydrated, e.g. freeze dried. When the dehydrated liposomes are to be used, rehydration is accomplished by adding an aqueous solution, e.g. distilled water or an appropriate buffer to the liposomes and allow them to rehydrate.

The liposomes of the present invention can be administered alone but will generally be administered together with a pharmaceutically acceptable carrier. The preparations may be administered parenterally, most preferably intravenously.

Since dosage regimens for busulphan are well known, the amount of the liposomal busulphan which is effective as therapeutic for the treatment of the above mentioned diseases or conditions in humans will be apparent to those skilled in the art.

This invention will now be described in greater detail by reference to the following non-limiting examples, in which:

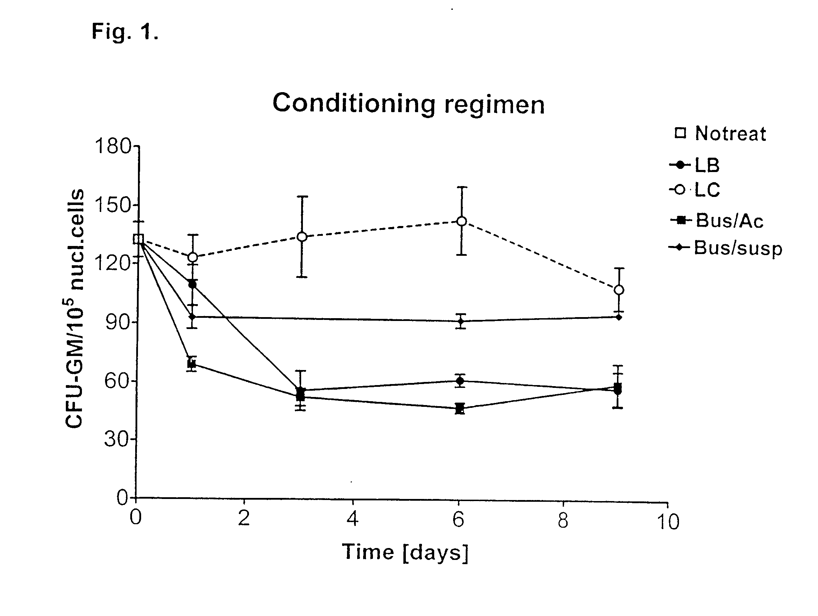

FIG. 1 shows myelosuppression kinetics of intravenously administered liposomal busulphan and orally administered busulphan after bone marrow transplantation conditioning regimen. Each data point represents the mean ±SD for 3 to 7 animals. All statistical calculations were made using median values.

### EXAMPLE 1

Preparation of liposome-encapsulated busulphan according to the most preferred embodiment of the invention comprising L-α-Phosphatidylcholine, 1,2-dioleolyl-sn-glycero-3-phosphate and cholesterol.

### Materials:

Busulphan (1,4-bis[methanesulphonoxy]butane) was obtained from Sigma Chemicals, St Louis, USA.
1,4 (¹⁴C-succinic acid) was purchased from Amersham, UK. Cholesterol, L-α-Phosphatidylcholine (egg, 100 mg/mL) and 1,2-Dioleolyl-sn-Glycero-3-phosphate (monosodium salt, 20 mg/mL) were obtained from Avanti Polar-Lipids Inc., Alabama USA.
Glucose- and sodium chloride solutions (50mg/mL and 9mg/mL, respectively) were obtained from Pharmacia & Upjohn, Sweden.

Liposomal busulphan was prepared using L-α-Phosphatidylcholine, 1,2-Dioleolyl-sn-Glycero-3-phosphate and cholesterol.
Lipids: L-α-Phosphatidylcholine (EPC), 1,2-Dioleolyl-sn-Glycero-3-phosphate (DOPA) and cholesterol in the molar ratio 9.45:1:9.4. were dissolved in chloroform. Busulphan was added. The mixture of lipids and busulphan was dried by evaporation to a thin film coating the inside of a round glass vessel. Any traces of solvent were removed under a gentle stream of nitrogen. The mixture was then hydrated with 25 mL of glucose solution (50mg/mL, pH 4.0) or 25 mL of sodium chloride solution (9mg/mL). Multilamellar vesicles were formed by vortexing the lipid-aqueous mixture for 10 minutes at room temperature. The suspension was transferred to an Extruder (LiposoFast 50, Avestin, Ottawa, Canada) and extruded under nitrogen, through 2 stacked polycarbonate filters of 100 nm pore size 5 times. All these preparations were performed under aseptic conditions. Busulphan concentrations were determined before and after filtration to determine the entrapment efficiency. The total phospholipid content was 16 mg/ mL.

Vesicle size distribution for the final liposomal preparation and the stability of liposomal preparation were studied over 20 days at + 4 °C using both dynamic light scattering (Malvern Autosizer) and laser diffraction (Malvern Mastersizer). The liposomal preparation was also examined for free crystals of busulphan using electron microscopy. The determination of busulphan concentrations of liposomes was performed using gas chromatography with electron capture detection (Hassan, M., Ehrsson, H. 1983. J Chromatogr 277:374.). These analyses showed that the formed liposomes were unilamellar vesicles with 220 ± 14 nm in diameter. The half-life of busulphan in the present formulation was determined to be 8.7 ± 2.7 days at + 4°C. The liposomes in the formulation were stable for 20 days at +4 °C, i.e. no aggregates of liposomes were observed as determined by dynamic light scattering and laser diffraction, nor were crystals of free busulphan observed as determined by electron microscopy. The busulphan concentration of the liposomes was 0.31 ± 0.03 mg/mL.

### EXAMPLE 2

Preparation of liposome-encapsulated busulphan according to a preferred embodiment of the invention comprising phosphatidylserine, phosphatidylcholine and cholesterol.

### Materials:

Phosphatidylserine was obtained from Avanti Polar-Lipids Inc., Alabama USA.
Otherwise as in Example 1.

This formulation of liposomal busulphan was prepared as in Example 1, but the lipids phosphatidylserine, phosphatidylcholine and cholesterol in a molar ratio of about 3:7:10, were used.

Analyses (as in Example 1) showed that the formed liposomes were unilamellar vesicles with 290±22 nm in diameter. The half-life of busulphan in the present formulation was determined to be 8 days at + 4°C. The liposomes in the formulation were stable for 23 days at +4°C, i.e. no aggregates of liposomes were observed as determined by dynamic light scattering and laser diffraction, nor were crystals of free busulphan observed as determined by electron microscopy. The busulphan concentration of the liposomes was 0.55±0.05 mg/mL.

### EXAMPLE 3

Preparation of liposome-encapsulated busulphan according to a preferred embodiment of the invention comprising cardiolipin, phosphatidylcholine and cholesterol.

### Materials:

Cardiolipin was obtained from Avanti Polar-Lipids Inc., Alabama USA. Otherwise as in Examples 1 and 2.

This formulation of liposomal busulphan was prepared as in Example 1, but the lipids cardiolipin, phosphatidylcholine and cholesterol, in a molar ratio of about 1:4:1.5 were used.

Analyses (as in Example 1) showed that the formed liposomes were unilamellar vesicles with 310±25 nm in diameter. The half-life of busulphan in the present formulation was determined to be 8 days at + 4°C. The liposomes in the formulation were stable for 18 days at +4°C, i.e. no aggregates of liposomes were observed as determined by dynamic light scattering and laser diffraction, nor were crystals of free busulphan observed as determined by electron microscopy. The busulphan concentration of the liposomes were 0.61±0.10 mg/mL.

### EXAMPLE 4

Preparation of liposome-encapsulated busulphan according to a preferred embodiment of the invention comprising phosphatidylcholine, cholesterol and stearoylamine.

### Materials:

Stearoylamine was obtained from Avanti Polar-Lipids Inc., Alabama USA. Otherwise as in Example 1.

This formulation of liposomal busulphan was prepared as in Example 1, but the lipids phosphatidylcholine, cholesterol and stearoylamine in a molar ratio of about 7:1:1 were used.

Analyses (as in Example 1) showed that the formed liposomes were unilamellar vesicles with 225±20 nm in diameter. The half-life of busulphan in the present formulation was determined to be 8 days at + 4°C. The liposomes in the formulation were stable for 14 days at +4°C, i.e. no aggregates of liposomes were observed as determined by dynamic light scattering and laser diffraction, nor were crystals of free busulphan observed as determined by electron microscopy. The busulphan concentration of the liposomes were 0.75±0.05 mg/mL.

### EXAMPLE 5

Biodistribution of liposome-encapsulated busulphan according to the invention, verifying decreased toxicity.

Distribution of intravenously administered liposomal busulphan, prepared according to Example 1 and hydrated with glucose, and free busulphan were studied using a C-14 labeled compound. The distribution of ¹⁴C-busulphan entrapped in liposomes was compared with an equal amount of free ¹⁴C-busulphan dissolved in DMSO: ethanol : propylene glycol in different organs. Rats were anaesthetized with ether, injected intravenously in the tail-vein with either liposomal ¹⁴C-busulphan (1.5 mL) or ¹⁴C-busulphan (O.25 mL) dissolved in (DMSO: ethanol: propylene glycol in the ratio 0.35:0.25: 0.40). The injections of free busulphan were followed by 1.25 mL of saline solution to compensate for the volume differences. At appropriate times (0.17, 0.5, 1, 2, 3, 4, 6, 8, 10, 12, 18, 24 hr) the animals (3 animals for each time/point) were anaesthetized with pentobarbital (50 mg/kg) and blood samples were withdrawn through heart puncture (3-4 mL). Plasma was immediately separated from blood cells at 4000 RPM and stored at - 20 °C until analysis. The animals were sacrificed under anesthesia and about 1 g of lungs, heart, liver, brain, spleen, kidney and testis were removed, washed from blood in isotonic saline solution and weighed. The marrow was removed from the femur by flashing with 1 mL of saline solution which was weighed before and after the flushing. The femur was also weighed before and after the flushing (as a control of the marrow weight). All organs were kept at - 20 °C before assay either for busulphan concentrations or for the radioactivity distribution. The distribution of the radioactivity which represents busulphan and its metabolites was studied in the organs by liquid scintillation. About 400 mg of each organ were minced and homogenized in 1 mL saline solution. Duplicates of the homogenized samples (0.4mL) were transferred into glass counting-vials and solubilized with 1 mL of Soluene-350 / isopropanol (1:1) for 2 h at room temperature. To decolourize the samples 0.2 mL 30% hydrogen peroxide was added after solubilization was completed. The vials were incubated with hydrogen peroxide at 50°C for 30 min. After cooling 15 mL of Hionic Fluor were added. The vials were kept at room temperature for 2 days to allow chemiluminescence to decay, and then counted for ¹⁴C-radioactivity.

The distribution of the total radioactivity in these organs is listed in table 1. The radioactivity for both administration forms were calculated as AUC for each organ (1 g) from time 0 to 18 hr after the treatment. As can be seen from table 1, a significant accumulation (p < 0.001) was observed for bone marrow (3 fold) and spleen (2 fold), the target organs for busulphan therapy, after the administration of L-Bu compared with that obtained after the administration of D-Bu. No accumulation in the liver, the main target organ known for busulphan toxicity, was observed. Also a significant (p<0.05) decrease was observed in the distribution of the radioactivity to the heart, lungs, brain and blood , i.e. organs also known to be susceptible for busulphan toxicity. Thus, the improved biodistribution of busulphan, when formulated according to the present invention, optimizes high dose busulphan therapy and decreases its toxicity.

### EXAMPLE 6

Comparison of the pharmacokinetic characteristics between liposome-encapsulated busulphan and busulphan in its free form.

Liposome-encapsulated busulphan (LB) was prepared according to Example 1, and hydrated with glucose. Pharmacokinetics was studied in a rat model in doses 0.5-3.5 mg/kg body weight. Means and standard deviations were calculated from the experimental data. The concentration-time curves were adjusted to the data sets via non-linear iterative least square regression analysis. Curve modeling was performed according to the classical One or two compartment open models. The pharmacokinetics parameters were calculated on a PC using WINNONLIN ver 1.5.

The symbols are:
- BW:: body weight (kg)
- C0:: intercept at time zero (µg/mL)
- ta:: distribution half-life (h)
- tβ:: elimination half-life (h)
- Vd:: distribution volume
- Cp:: Concentration in plasma
- Cl tot:: total body clearance,
- Cbm:: Concentration in bone marrow
- AUCO:: area under the concentration-time curve, estimated with the trapezoidal rule (µg/mL.h).
The statistical analysis (F-test, Student's t-test and variance analysis) was performed using stat-mate (Graph-Pad, version 1.0).

The busulphan in liposomal form was well tolerated from 0.5- 3.5 mg/ kg. All relevant pharmacokinetic parameters for L-Bu and D-Bu are listed in table 2.

Peak plasma levels were within the range from 833 - 3538 ng/mL after the administration of D-Bu which is higher than that obtained after the administration of L-Bu (range 1459- 2603 ng/mL). Busulphan pharmacokinetics were linear within the range 0.5- 3.5 mg/kg for both D-Bu and for L-Bu. The bioavailability for L-Bu as compared with D-Bu (table 2) was 0.85. However, the distribution of liposomal busulphan to the bone marrow was significantly (p<0.05) higher with a ratio 1.6 of AUC marrow/AUC blood compared to 0.90 obtained after the administration of D-Bu.

The distribution volumes of L-Bu were significantly higher (table 2) compared with those for D-Bu (mean: 1.39 and 0.67 L/kg, respectively). It can also be seen that the apparent distribution volume (Vd x Cp/ CBm) in bone marrow (which reflects the concentration of the drug in bone marrow compared to the concentration of busulphan in plasma) is significantly lower after the administration of L-Bu compared with D-Bu. As it is shown in table 2, the elimination half-lives were significantly longer in blood and marrow after the administration of L-Bu compared with those obtained after D-Bu. No significant differences were observed in clearance for L-Bu or D-Bu. Thus, liposome-encapsulted busulphan according to the invention exhibits linear pharmacokinetcs.

**Table 2**

| **Parameter** | **Blood (DMSO)** | **BM (DMSO)** | **Blood (Lipo)** | **BM (Lipo)** |
|---|---|---|---|---|
| AUC (µg.hr/mL) | 11.82±2.22 | 9.85±0.67 | 9.93±0.15 | 15.82±2.25 |
| t1/2 (hr) | 1.75±0.37 | 1.53±0.01 | 2.52±0.09 | 3.08±1.06 |
| V (L/kg) | 0.68±0.06 | *0.80±0.10 | 1.39±0.22 | *0.44±0.05 |
| CL (mLmin-¹) | 0.072±0.004 | 0.66±0.01 | 0.099±0.02 | 0.055±0.03 |

### EXAMPLE 7

Comparison of myelo-suppressive effect between orally administered busulphan and intravenously administered liposomal busulphan according to the invention.

Liposome-encapsulated busulphan (LB) was prepared according to Example 1, and hydrated with glucose. Busulphan for oral administration was either suspended in water (Bus/susp) or diluted in acetone as a stock solution which was mixed with water before administration (Bus/Ac). The control group for CFU-GM experiments consists of untreated animals and animals treated by blank preparations containing liposomal solution without busulphan (LC).

Liposomal busulphan was administered through tail vein while the oral forms were administered through gastric tube. The treatment was a conditioning regimen-like schedule with busulphan administration twice a day for 4 days. All doses are presented as mg/ kg ± SD. The total doses of busulphan were: LB (20.0 ± 2.0), Bus/ susp (21.0 ± 2.1) and Bus/Ac (21.5 ± 1.1).

Bone marrow was harvested on day 1, 3, 6 or 9 after the last dose of the conditioning regimen. Each treatment or control group consisted of a minimum of three animals at each time point.

Mice were sacrificed under general anaesthesia with ether. Both femurs were removed and bone marrow was flushed out under sterile conditions. CFU-GM assay was performed in MethoCult M3530. Briefly: The red cells were lysed with ammonium chloride solution for 10 minutes on ice. Cells were washed twice in PBS and cell number and viability were determined by trypan blue exclusion. Nucleated cells (1x10⁵) were plated in 1.1 ml of MethoCult M3530 in 35 mm Petri dishes in triplicates. The plates were placed in 37 °C, 5% CO₂ and 80% humidity incubator. Colonies > 50 cells were counted on the day 7 using an inverted microscope.

As it can be seen in Fig 1, all formulations of busulphan showed a significant decrease in CFU-GM compared with untreated animals. The effect of LB was similar to that obtained after the administration of Bus/Ac. A significant decrease after both treatments was reached at day 3 (40-50% compared with untreated animals) and continued to day 6 and 9. However, a significant difference was observed at the first day of treatment, where the effect of Bus/Ac was higher (expressed as the number of CFU-GM) on the bone marrow compared with that obtained after LB (55% compared to 90%, respectively). Both LB and Bus/Ac had a higher and significant effect on the bone marrow compared with that of busulphan in suspension. The CFU-GM number obtained after treatment with busulphan in suspension was about 75% compared to that obtained in the control group. Mann-Whitney test showed significant p-values when untreated animals were compared with all busulphan treated groups (except LB on day 1). Kruskal-Wallis test showed significant differences among treatment groups compared at the same time point (day 1 p=0.02, day 3 p=0.01, day 6 p=0.0004 and day 9 p=0.008). The administration of liposomes to the mice did not influence the colony forming capacity of the bone marrow and no significant difference was found compared with the CFU-GM obtained from the control group (p> 0.05).

Thus, this example demonstrates that no side effects were seen when liposomes were administered alone and that myelo-suppression mediated by the intravenously administered liposomal formulation of busulphan was well comparable with orally administered busulphan, suggesting that the liposomal formulations of busulphan according to the invention are well suited for clinical use.

### EXAMPLE 8

Studies of pharmacokinetics when liposome encapsulated busulphan is administered to humans.

Eleven patients were included: five children and six adults. The patients and diagnosis are shown in table 3. The patients received a single dose of liposomal busulfan (2-9 mg). The dose was given one day before their ordinary treatment. The liposomal dose was given as a short infusion (20-40 min).

The liposomal form used in the present study, as well as the method for its manufacture are described in Example 1. i.e. the liposomes have a diameter of 200 nm and the composition is the following: L-α-phosphatidylcholine, 1,2-dioleolyl-sn-glycero-3-phosphate and cholesterol in the molar ratio 9.45:1:9.4.

**Table 3**

| Pat no. | Ace, years | Diagnosis | Dose, mg | AUC | T½, hours | Clearance, L/kg |
|---|---|---|---|---|---|---|
| 1 A | 44 | AML | 6.60 | 567 | 2.21 | 0.16 |
| 2 A | 52 | MDS | 4.90 | 466 | 2.87 | 0.18 |
| 3 A | 33 | AML | 3.60 | 481 | 1.88 | 0.15 |
| 4 A | 57 | CML | 7.30 | 486 | 3.21 | 0.18 |
| 5 A | 55 | MDS | 6.95 | 383 | 3.19 | 0.21 |
| 6 A | 31 | AML | 8.20 | 706 | 2.48 | 0.16 |
| | | | | | | |
| 1 C | 0.83 | SCID | 1.90 | 2621 | 2.23 | 0.15 |
| 2 C | 13 | GCD | 4.84 | 1001 | 3.51 | 0.14 |
| 3 C | 9 | MDS | 5.20 | 2135 | 3.36 | 0.12 |
| 4 C | 8 | AML | 5.15 | 1021 | 2.12 | 0.19 |
| 5 C | 16 | AML | 7.72 | 552 | 2.26 | 0.20 |

The present study and the pharmacokinetic analysis showed that:
- there is not any significant difference between adult and children in drug clearance (low dose)
- no differences were observed in elimination half life
- no allergic reaction was observed in any of the patients
- the formulation is well tolerated for human use
- no sign of local toxicity was seen during the administration

### REFERENCES CITED

Oakhill et al. 1981. J. Clin.Pathol. 34(5):495-500.
Canellos G.P., Chronic Leukemias. In: Cancer: Principles and Practice of Oncology, 2^{nd} Edition. 1985, DeVita V.T. Jr et al., (Eds). J.B. Lippinicott Co. Philadelpha, Pa. pp 1739-1752.
Santos, G.W. et al. 1983. N. Engl. J. Med. 309:1347-1353.
Lu, C. et al. 1984. Cancer Treatm. Repts. 68:711-717.
Miller, C. et al. 1991. Blood 78:1155.
Vassal, G. et al. 1990. Cancer Res. 50:6203-6207.
Bandini, G. et al. 1994. Bone Marrow Transpl. 13:577-581.
Bhagwatwar H.P, Phadungpojna S, Chow D.S,Andersson B.S. 1996. Cancer Chemother Pharmacol 37:401.
Ehninger G, Schuler U, Renner U, Ehrsam M, Zeller K.P, Blanz J, Storb R, Deeg H.J. 1995. Blood 85:3247.
Kennedy Jr G.L,Sherman H. 1986. Drug and Chemical Toxicology 9:147.
Kinney L.A, Burgess B.A, Stula E.F,Kennedy Jr G.L. 1993. Drug and Chemical Toxicology 16:175.
Malley L.A, Slone Jr T.W, Makovec G.T, Elliott G.S,Kennedy Jr G.L. 1995. Fundam Appl Toxicol 28:80.
Martino M, Morabito F, Messina G, Irrera G, Pucci G,Iacopino P. 1996. Haematologica 81:59.
Sperling S,Larsen I.G. 1979. Acta Ophthalmol (Copenh) 57:891.
Yellowlees P, Greenfield C,McIntyre N. 1980. Lancet 2:1004.
Shuler et al. 1997. Abstract EBMT-meeting, Chamonix, France.
Bangham et al. 1965. J. Mol. Biol. 13:238-252.
Papahadjopoulos et al. Biochim. Biophys. Acta. 1967. 135:624-638.
Hassan, M., Ehrsson, H. 1983. J Chromatogr 277:374.
U.S. patent 5,430,057
U.S. patent 5,559,148

## Claims

1. A liposome composition comprising busulfan encapsulated in liposomes having a size of from 50 to 300 nm, for use in a myelosuppressive treatment.

2. A liposome composition according to claim 1 wherein the lipid is selected from phosphatidylcholine, phosphatidylserine, glycero-3-phosphate, cholesterol, cardiolipin, stearoylamine, dicetylphosphate and phosphatidylglycerol.

3. A liposome composition according to claim 2, comprising L-α-phosphatidylcholine, 1,2-dioleolyl-sn-glycero-3-phosphate and cholesterol.

4. A liposome composition according to claim 3, comprising L-α-phosphatidylcholine, 1,2-dioleolyl-sn-glycero-3-phosphate and cholesterol in a molar ratio of 10:1:10.

5. A liposome composition according to claim 2, comprising phosphatidylserine, phosphatidylcholine and cholesterol.

6. A liposome composition according to claim 5, comprising phosphatidylserine, phosphatidylcholine and cholesterol in a molar ratio of 3:7:10.

7. A liposome composition according to claim 2, comprising cardiolipin, phosphatidylcholine and cholesterol.

8. A liposome composition according to claim 7, comprising cardiolipin, phosphatidylcholine and cholesterol in a molar ratio of 1:4:1.5.

9. A liposome composition according to claim 2, comprising phosphatidylcholine, cholesterol and stearoylamine.

10. A liposome composition according to claim 9, comprising phosphatidylcholine, cholesterol and stearoylamine, in a molar ratio of 7:1:1.

11. A liposome composition according to any of the above claims, wherein the molar ratio of busulfan to lipid is from 1:2 to 1:140.

12. A liposome composition according to any of the above claims, wherein the liposomes are unilamellar.

13. A liposome composition according to any of the above claims, **characterized in that** the liposomes are in a dehydrated state.

14. A method of preparing a liposome composition according to any of the claims 1-12, comprising the steps of
(i) dissolving the lipid(s) in an organic solvent medium to obtain an organic solvent lipid solution and adding busulfan to the organic solvent lipid solution;
(ii) drying the organic solvent lipid solution to a film;
(iii) rehydrating the film by use of a suitable hydrating agent;
(iv) producing liposomes by applying suitable liposome forming treatment to the film; and
(v) extruding the liposomes through filtering means suitable for providing a liposome composition having a liposome size of from 50 to 300 nm

15. A method according to claim 14 wherein the hydrating agent in step (iii) is aqueous NaCl or aqueous glucose.

16. A method according to claim 14 or 15 for preparing a liposome composition according to claim 13, **characterised in that** a dehydration is performed on the liposomes obtained in step (v).

17. A liposome composition according to any of the claims 1-13 for parenteral administration.

18. A liposome composition according to claim 17 for intravenous administration.

## Patentansprüche

1. Liposomzusammensetzung umfassend in Liposomen mit einer Größe von 50 bis 300 nm eingekapseltes Busulfan zur Verwendung in einer myelosuppressiven Behandlung.

2. Liposomzusammensetzung nach Anspruch 1, wobei das Lipid ausgewählt ist aus Phosphatidylcholin, Phosphatidylserin, Glycero-3-phosphat, Cholesterol, Cardiolipin, Stearoylamin, Dicetylphosphat und Phosphatidylglycerol.

3. Liposomzusammensetzung nach Anspruch 2, umfassend L-α-Phosphatidylcholin, 1,2-Dioleolyl-sn-glycero-3-phosphat und Cholesterol.

4. Liposomzusammensetzung nach Anspruch 3, umfassend L-α-Phosphatidylcholin, 1,2-Dioleolyl-sn-glycero-3-phosphat und Cholesterol in einem Molverhältnis von 10:1:10.

5. Liposomzusammensetzung nach Anspruch 2, umfassend Phosphatidylserin, Phosphatidylcholin und Cholesterol.

6. Liposomzusammensetzung nach Anspruch 5, umfassend Phosphatidylserin, Phosphatidylcholin und Cholesterol in einem Molverhältnis von 3:7:10.

7. Liposomzusammensetzung nach Anspruch 2, umfassend Cardiolipin, Phosphatidylcholin und Cholesterol.

8. Liposomzusammensetzung nach Anspruch 7, umfassend Cardiolipin, Phosphatidylcholin und Cholesterol in einem Molverhältnis von 1:4:1,5.

9. Liposomzusammensetzung nach Anspruch 2, umfassend Phosphatidylcholin, Cholesterol und Stearoylamin.

10. Liposomzusammensetzung nach Anspruch 9, umfassend Phosphatidylcholin, Cholesterol und Stearoylamin in einem Molverhältnis von 7:1:1.

11. Liposomzusammensetzung nach einem der vorstehenden Ansprüche, wobei das Molverhältnis von Busulfan zu Lipid 1:2 bis 1:140 beträgt.

12. Liposomzusammensetzung nach einem der vorstehenden Ansprüche, wobei die Liposomen unilamellar sind.

13. Liposomzusammensetzung nach einem der vorstehenden Ansprüche, **dadurch gekennzeichnet, dass** die Liposomen in einem dehydratisierten Zustand vorliegen.

14. Verfahren zum Herstellen einer Liposomzusammensetzung nach einem der Ansprüche 1-12, umfassend die Schritte
(i) Auflösen des Lipids (der Lipide) in einem organischen Lösungsmittelmedium, um eine Lösung von Lipid in organischem Lösungsmittel zu erhalten, und Zugeben von Busulfan zu der Lösung von Lipid in organischem Lösungsmittel.
(ii) Trocknen der Lösung von Lipid in organischem Lösungsmittel zu einem Film;
(iii) Rehydratisieren des Films unter Verwendung eines geeigneten Hydratisierungsmittels;
(iv) Erzeugen von Liposomen durch Anwenden einer geeigneten Liposombildungsbehandlung auf den Film; und
(v) Extrudieren der Liposomen durch Filtereinrichtungen, die sich zum Bereitstellen einer Liposomzusammensetzung mit einer Liposomgröße von 50 bis 300 nm eignen.

15. Verfahren nach Anspruch 14, wobei das Hydratisierungsmittel in Schritt (iii) wässriges NaCl oder wässrige Glucose ist.

16. Verfahren nach Anspruch 14 oder 15 zum Herstellen einer Liposomzusammensetzung nach Anspruch 13, **dadurch gekennzeichnet, dass** eine Dehydratisierung an den in Schritt (v) erhaltenen Liposomen durchgeführt wird.

17. Liposomzusammensetzung nach einem der Ansprüche 1-13 für eine parenterale Verabreichung.

18. Liposomzusammensetzung nach Anspruch 17 für eine intravenöse Verabreichung.

## Revendications

1. Composition de liposome comprenant du busulfan encapsulé dans des liposomes ayant une taille allant de 50 nm à 300 nm, pour une utilisation dans un traitement myélosuppressif.

2. Composition de liposome selon la revendication 1, dans laquelle le lipide est choisi parmi la phosphatidylcholine, la phosphatidylsérine, le glycéro-3-phosphate, le cholestérol, le cardiolipide, la stéaroylamine, le dicétylphosphate et le phosphatidylglycérol.

3. Compositions de liposome selon la revendication 2, comprenant la L-α-phosphatidylcholine, le 1,2-dioléolyl-sn-glycéro-3-phosphate et le cholestérol.

4. Composition de liposome selon la revendication 3, comprenant la L-α-phosphatidylcholine, le 1,2-dioléolyl-sn-glycéro-3-phosphate et le cholestérol dans un rapport molaire de 10:1:10.

5. Composition de liposome selon la revendication 2, comprenant la phosphatidylsérine, la phosphatidylcholine et le cholestérol.

6. Composition de liposome selon la revendication 5, comprenant de la phosphatidylsérine, de la phosphatidylcholine et du cholestérol dans un rapport molaire de 3:7:10.

7. Composition de liposome selon la revendication 2, comprenant du cardiolipide, de la phosphatidylcholine et du cholestérol.

8. Composition de liposome selon la revendication 7, comprenant du cardiolipide, de la phosphatidylcholine et du cholestérol dans un rapport molaire de 1:4:1,5.

9. Composition de liposome selon la revendication 2, comprenant de la phosphatidylcholine, du cholestérol et de la stéaroylamine.

10. Composition de liposome selon la revendication 9, comprenant de la phosphatidylcholine, du cholestérol et de la stéaroylamine, dans un rapport molaire de 7:1:1.

11. Composition de liposome selon l'une quelconque des revendications précédentes, dans laquelle le rapport molaire busulfan/lipide va de 1:2 à 1:140.

12. Composition de liposome selon l'une quelconque des revendications précédentes, dans laquelle les liposomes sont unilamellaires.

13. Composition de liposome selon l'une quelconque des revendications précédentes, **caractérisée en ce que** les liposomes sont à l'état déshydraté.

14. Procédé de préparation d'une composition de liposome selon l'une quelconque des revendications 1 à 12, comprenant les étapes consistant à :
(i) dissoudre le(s) lipide(s) dans un milieu de solvant organique pour obtenir une solution de lipide(s) dans un solvant organique et ajouter du busulfan à la solution de lipide(s) dans un solvant organique ;
(ii) sécher la solution de lipide(s) dissolvant la matière organique en un film ;
(iii) réhydrater le film en utilisant un agent d'hydratation approprié ;
(iv) produire des liposomes en appliquant le traitement de formation de liposomes approprié au film ; et
(v) extruder les liposomes à travers de moyens de filtration appropriés pour fournir une composition de liposomes ayant une taille de liposomes allant de 50 nm à 300 nm.

15. Procédé selon la revendication 14, dans lequel l'agent d'hydratation de l'étape (iii) est NaCl aqueux ou le glucose aqueux.

16. Procédé selon la revendication 14 ou 15, pour la préparation d'une composition de liposomes selon la revendication 13, **caractérisé en ce qu'**une déshydratation est réalisée sur les liposomes obtenus à l'étape (v).

17. Composition de liposomes selon l'une quelconque des revendications 1 à 13, pour une administration parentérale.

18. Composition de liposomes selon la revendication 17, pour une administration intraveineuse.
